# EUROPÄISCHE PATENTSCHRIFT

(11) **EP 3 597 267 B1**
(45) Veröffentlichungstag und Bekanntmachung des Hinweises auf die Patenterteilung: **20.10.2021**
(21) Anmeldenummer: 19174825.0
(22) Anmeldetag: 16.05.2019
(51) Int. Cl.: A61N 1/375

(54) **GLAS-METALL-DURCHFÜHRUNG**
GLAS-METAL FEEDTHROUGH
TRAVERSÉE EN VERRE ET EN MÉTAL

(30) Priorität: 20.07.2018 DE 102018005733
(43) Veröffentlichungstag der Anmeldung: 22.01.2020
(60) Teilanmeldung: 21193032.6
(73) Patentinhaber: Schott AG, 55122 Mainz (DE)
(72) Erfinder: Hettler, Robert, 84036 Kumhausen (DE)
(74) Vertreter: Sawodny, Michael-Wolfgang

(56) Entgegenhaltungen:
- EP-A1- 1 028 923
- DE-A1- 19 842 943
- US-A1- 2011 031 698

## Beschreibung

Die Erfindung betrifft eine Glas-Metall-Durchführung, bestehend aus einem Außenleiter, einem Glasmaterial und einem Innenleiter sowie die Verwendung in einem medizinischen, insbesondere einem implantierbar medizinischen Gerät und Vorrichtungen sowie Geräten mit einer derartigen Glas-Metall-Durchführung .

Glas-Metall-Durchführungen sind in verschiedenen Anwendungsbereichen der Elektrotechnik eingesetzt. Beispielsweise werden diese für die Durchführung elektrischer Leiter in Gehäusen für Bauelemente in der Elektronik und der Sensorik verwandt. In diesem Zusammenhang weisen derartige Bauteile Schmelzverbindungen von Gläsern mit verschiedenen Metallmaterialien auf. Durch das Einschmelzen eines Innenleiters aus Metall in ein Glas- oder Glaskeramikmaterial, das von einem Außenleiter aus Metall umschlossen wird, kann eine hermetisch dichte Durchführung eines Leiters in ein Gehäuse zur Verfügung gestellt werden. Ganz besondere Glas-Metall-Durchführungen sind solche Durchführungen, bei denen die Durchführung selbst bzw. Teile der Durchführung in Kontakt mit dem menschlichen Körper kommt. Bei derartigen Durchführungen kommt es darauf an, dass eine hohe Korrosionsfestigkeit sowie eine gute Langzeitbeständigkeit aller eingesetzter Komponenten gewährleistet werden. Insbesondere sollen derartige Durchführungen nur begrenzte Mengen an Ni abgeben, um die Bildung von allergischen Reaktionen zu verhindern. Dies ist definiert in den Normen für die Grenzwerte der sogenannten Nickellässigkeit. Diesbezüglich wird auf die DIN EN1811 und DIN EN12472 verwiesen.

Bei Glas-Metall-Durchführungen werden als Materialien für den Durchführungsleiter vorwiegend Fe-Ni-, Fe-Ni-Co-, Fe-Ni-Cr-Legierungen eingesetzt. Der Vorteil dieser Materialien ist deren exzellente Anpassung der thermischen Dehnung an die Einschmelzgläser. Allerdings haben all diese Materialien signifikante Anteile an Ni im Grundmaterial. Darüber hinaus weisen sie Nickel-Beschichtungen auf, um die Materialien vor Korrosion zu schützen, die wiederum Nickel in unerwünschter Menge freisetzen.

Um eine Freisetzung von Ni zu verhindern, wurde im Stand der Technik vorgesehen, die Durchführungsleiter bzw. Innenleiter mit einer genügend dicken Goldschicht zu belegen, um die Nickeldurchdringung zu reduzieren. Dies hatte allerdings den Nachteil, dass es nur unzureichend gelingt, den Austritt von Ni zu verhindern, oder um dies zu erreichen, sehr dicke Au-Schichten mit einer Dicke von mehr als 2,5 µm nötig waren.

Alternativ zu einer Lösung mit beschichteten Leitern schlägt die DE 198 42 943 A1 als nächstliegender Stand der Technik vor, als Innenleiter Tantal zu verwenden oder alternativ nickelfreien, rostfreien und ferritischen Edelstahl wie beispielsweise den Stahl gemäß US-Norm AISI446, bei dem es sich um einen nicht rostenden, hitzebeständigen ferritischen Chrom-Stahl mit Aluminiumzusatz handelt. Der Ausdehnungskoeffizient des ferritischen Edelstahles AISI446 liegt nur wenig höher als bei gebräuchlichen Gläsern. Daher bestand die Gefahr, dass der Innenleiter sich beim Abkühlen stärker als das Glas zusammenzieht und die Schnittstelle zum Glas aufreißt. Ein weiterer Nachteil der DE 198 42 943 A1 ist, dass die Auswahl von Ni-freien Materialien begrenzt war, da eine ausreichende Dichtigkeit der Durchführung nur dann zur Verfügung gestellt wird, wenn der Ausdehnungskoeffizient des Innenleiter αᵢₙₙₑₙ geringer war als der Ausdehnungskoeffizient des Glases α_{Glas.}

Aus der US 3,770,568 A ist eine Glaszusammensetzung bekannt geworden, die für hermetisch dichte Tantal-Elektrolyt-Kondensatoren eingesetzt werden kann und einen effektiven Chromanteil in der Glaszusammensetzung umfasst. Das Dokument EP-A-1 028 923 offenbart eine Glas-Metall-Durchführung gemäß Oberbegriff des Anspruchs 1.

Aufgabe der Erfindung ist es somit, eine Glas-Metall-Durchführung anzugeben, die eine breitere Auswahl an Materialien für den Innenleiter zulässt und eine dichte Durchführung zur Verfügung stellt.

Insbesondere sollen Innenleiter möglich sein, die einerseits kein Nickel freisetzen, wenn sie mit dem menschlichen Körper in Berührung kommen, andererseits aber auch über eine ausreichende Verschweißbarkeit verfügen.

Diese Aufgabe wird gelöst durch eine Glas-Metall-Durchführung mit den Merkmalen des Patentanspruchs 1 in seiner Gesamtheit. Vorteilhafte Ausgestaltungen der Erfindung sind Gegenstand der Unteransprüche. Ausführungen, die nicht dem Gegenstand des Anspruchs 1 entsprechen, sind nicht Teil der Erfindung.

Eine erfindungsgemäße Glas-Metall-Durchführung zeichnet sich dadurch aus, dass der Ausdehnungskoeffizient des Innenleiters α_{Innen} größer ist als der Ausdehnungskoeffizient des Glases α_{Glas} und der Unterschied zwischen dem Ausdehnungskoeffizienten des Außenleiters α_{außen} und dem Ausdehnungskoeffizienten des Glases α_{Glas} mindestens 2 ppm/K, bevorzugt mindestens 4 ppm/K im Temperaturbereich 20° C bis zur Glastransformationstemperatur beträgt und der Ausdehnungskoeffizient des Außenleiters α_{außen} größer ist als der Ausdehnungskoeffizient α_{Glas} des Glases. Durch die Druckvorspannung des Außenleiters auf das Glas wird erreicht, dass das Glas auch einen positiven Druck (Fugendruck) auf das Glas zu Innenleiter Interface ausübt. Hieraus ergibt sich der Vorteil, dass dieser Druck die Verbindung zuverlässig dicht hält. Weiter ist eine geringe Bauhöhe der Glas-Metall-Durchführung erreicht und diese für unterschiedliche Anwendungsbereiche, wie Geräte zur Überwachung von Patienten oder Armbänder im Freizeitbereich, einsetzbar.

Die Einschränkung auf eine größere Dehnung des Pins als Innenleiter gegenüber Glas und eine um mindestens 2 ppm/K größere Dehnung des Aussenleiters gegenüber Glas ist insbesondere bei hoch dehnenden Materialien für den Pin mit einer Dehnung oberhalb von 10 ppm/K von großem Effekt. In diesem Bereich sind kaum gute Einschmelzgläser verfügbar.

Überraschender Weise haben die Erfinder herausgefunden, dass beispielsweise der Einsatz von ferritischem Edelstahl mit einem hohen Chromanteil zwischen 10,5 und 20 %, bevorzugt ein Edelstahl mit einem Chromanteil von 15 bis 17 %, beispielsweise ferritischem Edelstahl AISI 430, möglich ist. Alternativ können zu den ferritischen Edelstählen auch andere Materialien wie Molybdän, Wolfram oder Platin verwandt werden. Diese weiteren Materialien haben ebenso ein niedriges Allergiepotential. Auch für diese Materialien muss die erfindungsgemäße Bedingung αᵢₙₙₑₙ > α_{Glas} für die Ausdehnungskoeffizienten gelten, wobei αᵢₙₙₑₙ der Ausdehnungskoeffizient des Innenleiters ist und α_{Glas} der Ausdehnungskoeffizient des Glases.

Problematisch bei der Verwendung dieser Stähle ist allerdings, dass deren thermischer Ausdehnungskoeffizient αᵢₙₙₑₙ mit 11,6 bis 11,5 10⁻⁶/K deutlich höher ist als der Ausdehnungskoeffizient des Glases α_{Glas,} der im Bereich 10,6 bis 6,1 10⁻⁶/K liegt. Überraschender Weise haben die Erfinder herausgefunden, dass trotz eines größeren αᵢₙₙₑₙ des Leiters, der oberhalb von α_{Glas} liegt, entgegen dem Stand der Technik, der vorschreibt, dass bei Glas-Metall-Durchführungen die thermische Ausdehnung des Innenleiters nicht höher sein darf als die des verwendeten Glases, um eine ausreichende Dichtheit zur Verfügung zu stellen, auch im Falle αᵢₙₙₑₙ größer als α_{Glas}, dann eine dichte Einglasung zu Verfügung gestellt wird, wenn ein positiver hoher Fugendruck, der vom Außenleiter auf das Glas ausgeübt wird, zur Verfügung gestellt wird. Wie die weiteren Untersuchungen gezeigt haben, ist es nicht ausreichend für die Dichtheit, wenn der Fugendruck nur positiv ist, vielmehr muss ein Fugendruck von größer 30 MPa, bevorzugt größer 50 MPa, insbesondere größer 100 MPa erreicht werden, um eine zuverlässige Einglasung zu erreichen.

Ein solch hoher Fugendruck wird bei einem αᵢₙₙₑₙ kleiner α_{Glas} dann zur Verfügung gestellt, wenn vom Außenleiter genügend Druckvorspannung auf das Glas aufgeprägt wird. Der sich dann ergebende Fugendruck zwischen Glas und Innenleiter stellt sich beim Abkühlen der Durchführung nach dem Einschmelzen ein. Ist dieser Fugendruck deutlich positiv, d.h. größer 30 MPa bzw. größer 50 MPa, insbesondere größer 100 MPa, so bleibt der Übergang zwischen Glas und Metall, d.h., der Übergang von Glas zum Innenleiter geschlossen und damit dicht, obwohl αᵢₙₙₑₙ geringer ist als α_{Glas}. Der Fugendruck hängt direkt vom Dehnungsunterschied ab. Des Weiteren sind auch noch Abhängigkeiten von der Erfindungsgeometrie denkbar. Der Fugendruck ist eine Flächenpressung. Der Fugendruck drückt aus, mit welcher Kraft pro Flächeneinheit ein erster Körper auf einen zweiten drückt.

Um den notwendigen Fugendruck aufzubringen, beträgt der Unterschied zwischen dem Ausdehnungskoeffizienten des Außenleiters α_{außen} und dem Ausdehnungskoeffizienten des Glases mindestens 2 ppm/K, bevorzugt mindestens 4 ppm/K, wobei der Ausdehnungskoeffizient höher ist als der Ausdehnungskoeffizient des Glases α_{Glas}

Um den notwendigen Druck des Außenleiters auf das Glasmaterial aufzubringen und Dichtheit zu garantieren, ist vorgesehen, dass der Außenleiter aus einem nickelfreien, rostfreien, chemisch beständigen Stahl (Edelstahl) besteht. Der Ausdehnungskoeffizient des Außenleiters ist größer als der des Glases, um den notwendigen Fugendruck zur Verfügung zu stellen.

Besonders bevorzugt ist es, wenn es sich bei dem Außenleiter um einen austenitischen Edelstahl, bevorzugt den Edelstahl 316L, handelt, der sich durch eine gute Verschweißbarkeit und einen hohen Ausdehnungskoeffizienten auszeichnet.

Neben dem nickelfreien, ferritischen Edelstahl, insbesondere dem nicht härtbaren ferritischen Edelstahl mit einem hohen Chromanteil, dessen Ausdehnungskoeffizient αᵢₙₙₑₙ größer als derjenige des Glasmaterials ist, ist es auch vorstellbar, Molybdän oder Wolfram oder Platin für den Innenleiter zu verwenden. Bevorzugt werden die Ausdehnungskoeffizienten so gewählt, dass ein Fugendruck am Innenleiter von mindestens 30 MPa, bevorzugt mindestens 50 MPa, insbesondere mindestens 100 MPa, zur Verfügung gestellt wird.

Neben der Durchführung stellt die Erfindung auch die Verwendung der erfindungsgemäßen Glas-Metall-Durchführung in implantierbaren medizinischen Geräten oder Vorrichtungen zur Verfügung sowie ein in den menschlichen oder tierischen Körper oder Zellkulturen enthaltend lebende biologische Zellen einbringbares oder an diesen anbringbares Element mit einer erfindungsgemäßen Glas-Metall-Durchführung, wobei der Außenleiter und der Innenleiter zumindest in deren Oberflächenbereichen, die im Betriebszustand in Kontakt mit dem menschlichen oder tierischen Körper kommen, aus Metallen bestehen, welche ein vermindertes Allergiepotential aufweisen. Bevorzugt bestehen sowohl Außenleiter als auch Innenleiter aus diesen Metallen.

Das Metall des Außenleiters und des Innenleiters können in Kontakt mit dem menschlichen oder tierischen Körper oder den Zellkulturen kommen und zeichnen sich dadurch aus, dass sie kein Nickel und/oder Chrom abscheiden.

Bevorzugt besteht das Metall des Außenleiters und des Innenleiters, zumindest deren Oberflächenbereichen, die im Betriebszustand in Kontakt mit dem menschlichen oder tierischen Körper oder biologischen Zellen der Zellkultur kommen, aus nickelfreien und/oder chromfreien Stählen.

Besonders geeignet als Metall des Innenleiters zumindest in den Oberflächenbereichen, die im Betriebszustand in Kontakt mit dem menschlichen oder tierischen Körper oder biologischen Zellen der Zellkultur kommen, sind Metalle ausgewählt aus der Gruppe der ferritischen Edelstähle sowie Platin, Platin/Iridium, Niob, Titan, Molybdän, Wolfram sowie Kombinationen derselben. Die ferritischen Edelstähle umfassen AISI 4xx-Stämme wie beispielsweise AISI 430.

Das Metall des Außenleiters zumindest in den Oberflächenbereichen, die im Betriebszustand in Kontakt mit dem menschlichen oder tierischen Körper oder biologischen Zellen der Zellkultur kommen, ist bevorzugt ausgewählt aus der Gruppe
AISI 316 L
AISI 430
AISI 630
sowie Kombinationen derselben. Weitere Materialien für die Außenleiter können aus der Gruppe der austenitischen Edelstähle AISI 3xx bzw. ferritischen Edelstähle AISI4xx ausgewählt werden.

Die Erfindung soll nachfolgend anhand der Figuren nochmals eingehender beschrieben werden.

Es zeigen:
- Fig. 1: eine schematische Darstellung einer Ausführungsform der Erfindung;
- Fig. 2a-2b: das Streudiagramm des Fugendrucks von Leiter und Glas.

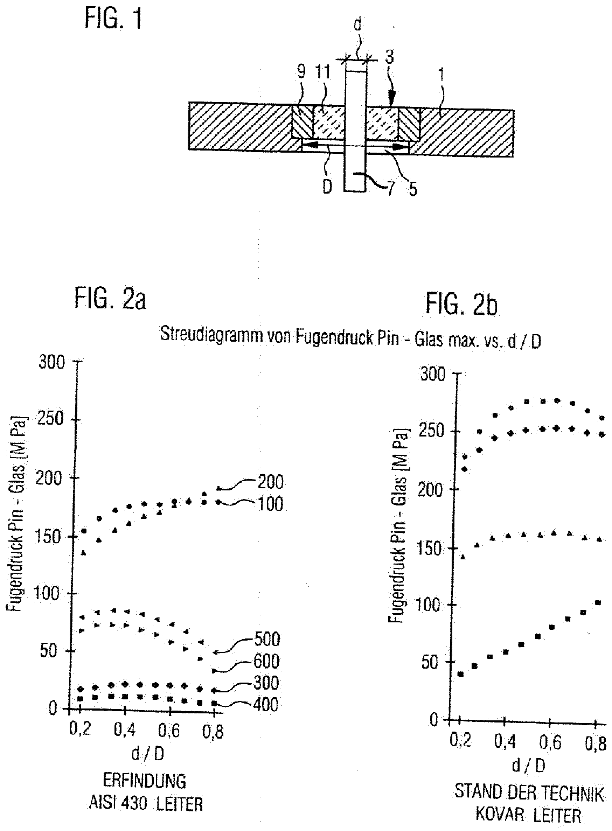

Figur 1 zeigt beispielhaft in schematischer Darstellung einen Schnitt durch eine Ausführungsform der Erfindung. Bei der in Figur 1 dargestellten Glas-Metall-Durchführung durch ein Gehäuse 1, das bevorzugt aus Aluminium hergestellt sein kann, ist eine Durchführung 3 in eine Öffnung 5 des Gehäuses 1 eingesetzt. Die Durchführung 3 umfasst einen Durchführungsleiter bzw. einen Innenleiter 7, der in einen Außenleiter 9 eingesetzt ist. Der Außenleiter 9 kann auch als Grundkörper bezeichnet werden und besteht bevorzugt aus einem austenitischen Edelstahl mit einem hohen thermischen Dehnungskoeffizienten. Der Außenleiter kann auch als Grundkörper bezeichnet werden und ist mit dem Gehäuse, bevorzugt durch Schweißen, verbunden. In dem Außenleiter ist zentriert der Innenleiter oder auch Durchführungsleiter 7 eingeglast. Dies geschieht dadurch, dass der Innenleiter 7 in einem isolierenden Glaskörper 11 den Raum innerhalb des Außenleiters 9 vollständig ausfüllt. Bei dem Glas, in dem der Innenleiter eingeschmolzen wird, handelt es sich bevorzugt um ein biokompatibles Glas.
Ebenfalls in Figur 1 dargestellt ist der Durchmesser d des Durchführungsleiters 7 sowie der sogenannte Lochdurchmesser D der Öffnung 5.

Erfindungsgemäß ist vorgesehen, dass das Glas einen Ausdehnungskoeffizient α_{Glas} aufweist, der Innenleiter einen Ausdehnungskoeffizient αᵢₙₙₑₙ und der Außenleiter einen Ausdehnungskoeffizient α_{außen}. Die Werkstoffe werden so gewählt, dass der Ausdehnungskoeffizient des Innenleiters αᵢₙₙₑₙ größer ist als der des Glases α_{Glas}. Der Unterschied zwischen dem Ausdehnungskoeffizient des Außenleiters und dem Ausdehnungskoeffizienten des Glases beträgt mindestens 2 ppm/K, bevorzugt mindestens 4 ppm/K. Der Ausdehnungskoeffizient des Außenleiters α_{außen} im Temperaturbereich von 20° C bis zur Transformationstemperatur ist größer als der Ausdehnungskoeffizient α_{Glas}. Hierdurch wird ein Fugendruck am Innenleiter von mindestens 30 MPa, bevorzugt von mindestens 50 MPa, insbesondere mindestens 100 MPa zur Verfügung gestellt.

Wenn, wie in der Erfindung gefordert, der Ausdehnungskoeffizient des Innenleiters αᵢₙₙₑₙ größer ist als derjenige des Glases α_{Glas}, so ergeben sich Probleme, eine ausreichende Dichtheit der Durchführung zur Verfügung zu stellen. In einem solchen Fall wird eine ausreichende Dichtigkeit nämlich nur dann gewährleistet, wenn der Außenleiter einen ausreichend hohen Fugendruck auf das Glas aufbauen kann. Den Fugendruck in Abhängigkeit von d/D gemäß dem Stand der Technik ist in Figur 2a gezeigt, wobei als Material für den Durchführungsleiter Kovar verwandt wurde, das mit einem α von 7,3 bis 6,6 10⁻⁶1/K leicht oberhalb des Wertes des Ausdehnungskoeffizienten α_{Glas} liegt und nicht von der Erfindung erfasst wird. Hierbei zeichnet d den Durchmesser des Leiters und D den Durchmesser der Öffnung 5. d/D gibt das Verhältnis zwischen Leiterdurchmesser d und Lochdurchmesser D an. Generell gilt, dass kleine Werte für d/D einen großen Spalt zwischen Leiter und Öffnung charakterisieren und große Werte von d/D einen kleinen Spalt zwischen Leiter und Öffnung.

Die Durchführungen gemäß Fig. 2a sind zwar hermetisch dicht, haben jedoch den Nachteil, dass Kovar-Legierungen einen hohen Nickelanteil haben, so dass Nickel aus den Durchführungsleiter freigesetzt werden kann.

Erfindungsgemäß ist daher vorgesehen, den Kovar-Durchführungsleiter durch ein Material zu ersetzen, das Nickel nicht freisetzt. Überraschend wurde herausgefunden, dass ein hierfür geeignetes Material ein ferritischer, Ni-freier Edelstahl, insbesondere AISI430, ist. Der Nachteil an dem Ni-freien, ferritischen Edelstahl, beispielsweise AISI430, ist jedoch, dass αᵢₙₙₑₙ bei 11,5 · 10⁻⁶ K⁻¹ liegt und damit deutlich oberhalb des Ausdehnungskoeffizienten α_{Glas} des Glasmaterials. Der Ausdehnungskoeffizient der eingesetzten Gläser α_{Glas} liegt nämlich in einem Bereich von 6,1 · 10⁻⁶ K⁻¹ bis 10,6 · 10⁻⁶ K⁻¹.

Um bei einer solchen Konstellation eine druckdichte Einglasung zu erreichen, muss ein ausreichend hoher Fugendruck, der vom Außenleiter aufgebracht wird, aufgebaut werden.

In Figur 2b ist der Fugendruck für derartige Durchführungen in Abhängigkeit von d/D gezeigt.

Wie aus Figur 2b hervorgeht, ergibt sich ein ausreichend hoher Fugendruck für die Verwendung von AISI 430 als Material für das Durchführungsbauteil, besonders dann, wenn der Außenleiter aus einem austenitischen Edelstahl mit einem α_{außen} von 18,3 10⁻⁶/K besteht. Ein derartiger Aufbau weist einen ausreichend hohen Fugendruck auf, um Dichtheit sicherzustellen. Der Fugendruck einer derartigen Materialkombination ist mit den Bezugsziffern 100 und 200 bezeichnet. Der Fugendruck steigt mit größerem d/D und damit kleine Spalte auf Werte oberhalb 150 MPa.

Die Kurven 300 und 400 beschreiben den Fugendruck für einen AISI 430 Durchführungsleiter, wobei der Außenleiter AISI 430 bzw. AISI 630 ist und das Glas einen Ausdehnungskoeffizienten von 10,6·10⁻⁶ aufweist und damit im Bereich des Ausdehnungskoeffizienten sowohl des Außenleiters wie des Durchführungsleiters liegt. Hierdurch kann der notwendige Fugendruck nicht aufgebaut werden. Generell zeigen derartige Materialkombinationen einen niedrigem Fugendruck auf. Die Kurven 300 und 400 verlaufen flach mit wenig Einfluss des Durchmesserverhältnisses.

Die Materialien der unterschiedlichen Kurven im Diagramm gemäß Figur 2b "Fugendruck über d/D" sind aus nachfolgender Tabelle zu entnehmen:

**Tabelle:**

| Kurve | Außen leiter Material | Außen leiter CTE bis TG | Glas Tg in °C | Glas CTE bis TG | Leiter Material | Leiter CTE bis TG | Symbol |
|---|---|---|---|---|---|---|---|
| 200 | AISI316L | 18,3 | 525 | 10,6 | AISI430 | 11,5 | ● |
| 100 | AISI316L | 18,4 | 565 | 6,1 | AISI430 | 11,6 | ▲ |
| 600 | AISI430 | 11,5 | 525 | 10,6 | AISI430 | 11,5 | ■ |
| 400 | AISI430 | 11,6 | 565 | 6,1 | AISI430 | 11,6 | ► |
| 300 | AISI630 | 11,4 | 525 | 10,6 | AISI430 | 11,5 | ◆ |
| 500 | AISI630 | 11,4 | 565 | 6,1 | AISI430 | 11,6 | ◄ |

Hierbei weisen die Materialkombinationen der Kurven 100, 200 einen besonders hohen Fugendruck auf, so dass hermetische Dichtheit der Durchführung zur Verfügung gestellt wird.

Die beschriebenen Glas-Metall-Durchführungen können in implantierbar medizinischen Geräten oder Vorrichtungen verwendet werden. Sie sind kostengünstig herstellbar und zeichnen sich durch einen sehr niedrigen Austritt von Ni aus. Des Weiteren weisen sie aufgrund des hohen Fugendruckes eine hermetische Dichtheit auf, d.h. eine Helium-Leckrate von weniger als 1 · 10⁻⁸mbarl/sec.

## Patentansprüche

1. Glas-Metall-Durchführung, bestehend aus einem Außenleiter (9), einem Glasmaterial (11) und einem Innenleiter (7), wobei der Innenleiter einen Ausdehnungskoeffizienten α_{Innen}, das Glas einen Ausdehnungskoeffizienten α_{Glas} und der Außenleiter einen Ausdehnungskoeffizienten α_{außen} aufweist, wobei der Ausdehnungskoeffizient des Innenleiters α_{Innen} größer ist als der Ausdehnungskoeffizient des Glases α_{Glas} und der Unterschied zwischen dem Ausdehnungskoeffizienten des Außenleiters α_{außen} und dem Ausdehnungskoeffizienten des Glases α_{Glas} mindestens 2 ppm/K, bevorzugt mindestens 4 ppm/K im Temperaturbereich 20° C bis zur Glastransformationstemperatur beträgt und der Ausdehnungskoeffizient des Außenleiters α_{außen} größer ist als der Ausdehnungskoeffizient α_{Glas} des Glases
**dadurch gekennzeichnet, dass** der Ausdehnungskoeffizient des Innenleiters α_{Innen} im Bereich 4 • α_{Glas} bis 1,5 • α_{Glas} liegt, wobei der Ausdehungskoeffizient αᵢₙₙₑₙ des Innenleiters bevorzugt doppelt so groß wie der Ausdehungskoeffizient α_{Glas} ist.

2. Glas-Metall-Durchführung nach Anspruch 1,
**dadurch gekennzeichnet, dass**
der Außenleiter aus einem bevorzugt nickelfreien, rostfreien, chemisch beständigen Stahl (Edelstahl) besteht.

3. Glas-Metall-Durchführung nach Anspruch 2,
**dadurch gekennzeichnet, dass**
der Außenleiter aus, rostfreiem chemisch beständigen Stahl, insbesondere austenitischen Edelstahl, insbesondere AISI 316 L, besteht.

4. Glas-Metall-Durchführung nach einem der Ansprüche 1 bis 3,
**dadurch gekennzeichnet, dass**
der Außenleiter aus nickelfreiem, rostfreiem, chemisch beständigem Stahl, bevorzugt einem ferritischen Edelstahl, bevorzugt AISI 430, besteht

5. Glas-Metall-Durchführung nach einem der Ansprüche 1 bis 4,
**dadurch gekennzeichnet, dass**
der Innenleiter aus einem der nachfolgenden Materialien besteht:
- nickelfreiem, ferritischen Edelstahl, insbesondere nicht härtbarem, ferritischem Edelstahl, bevorzugt mit einem hohen Chromanteil, insbesondere zwischen 10,5 und 20%, bevorzugt 15 und 17%, bevorzugt AISI 430
- Molybdän
- Wolfram
- Platin.
- Niob
- Titan
- Platin / Iridium

6. Glas-Metall-Durchführung nach einem der Ansprüche 1 bis 5,
**dadurch gekennzeichnet, dass**
die Ausdehnungskoeffizienten von Innenleiter und Außenleiter derart gewählt werden, dass ein Fugendruck am Innenleiter von mindestens 30 MPa, bevorzugt von mindestens 50 MPa, insbesondere mindestens 100 MPa aufgebaut wird.

7. Verwendung der Glas-Metall-Durchführung nach einem der Ansprüche 1 bis 6 in implantierbaren medizinischen Geräten oder Vorrichtungen.

8. In den menschlichen oder tierischen Körper oder Zellkulturen enthaltend lebende biologische Zellen einbringbares oder an diesen anbringbares Element mit einer Glas-Metall-Durchführung nach einem der Ansprüche 1 bis 6, wobei der Außenleiter und der Innenleiter zumindest in deren Oberflächenbereichen, die im Betriebszustand in Kontakt mit dem menschlichen oder tierischen Körper kommen, aus Metallen bestehen, welche ein vermindertes Allergiepotential aufweisen.

9. Element nach Anspruch 8,
**dadurch gekennzeichnet, dass**
das Metall des Außenleiters und des Innenleiters in Kontakt mit dem menschlichen oder tierischen Körper oder den Zellkulturen kein Nickel und/oder Chrom abscheidet.

10. Element nach mindestens einem der Ansprüche 8 bis 9,
**dadurch gekennzeichnet, dass**
das Metall des Außenleiters und des Innenleiters zumindest in deren Oberflächenbereichen, die im Betriebszustand in Kontakt mit dem menschlichen oder tierischen Körper oder biologischen Zellen der Zellkultur kommen, aus nickelfreien und/oder chromfreien Stählen besteht.

11. Element nach mindestens einem der Ansprüche 8 bis 10,
**dadurch gekennzeichnet, dass**
das Metall des Innenleiters zumindest in de Oberflächenbereichen, die im Betriebszustand in Kontakt mit dem menschlichen oder tierischen Körper oder biologischen Zellen der Zellkultur kommen, ausgewählt ist aus der Gruppe
- der ferritischen Edelstähle (AISI 4xx)
- Platin
- Platin/Iridium
- Niob
- Titan
- Molybdän
- Wolfram
sowie Kombinationen derselben

12. Element nach Anspruch 11,
**dadurch gekennzeichnet, dass**
das Metall des Außenleiters zumindest in den Oberflächenbereichen, die im Betriebszustand in Kontakt mit dem menschlichen oder tierischen Körper oder biologischen Zellen der Zellkultur kommen, ausgewählt ist aus der Gruppe
- der austenitischen Edelstähle AISI 3xxx, insbesondere
- AISI 316 L
- Der ferritischen Edelstähle AISI 4xxx, insbesondere
- AISI 430
- AISI 630
sowie Kombinationen derselben.

## Claims

1. A glass-metal feedthrough, consisting of an outer conductor (9), a glass material (11), and an inner conductor (7), wherein the inner conductor has a coefficient of expansion α_{Innen}, the glass has a coefficient of expansion α_{Glas}, and the outer conductor has a coefficient of expansion α_{auβen}, wherein the coefficient of expansion of the inner conductor αᵢₙₙₑₙ is greater than the coefficient of expansion of the glass α_{Glas} and the difference between the coefficient of expansion of the outer conductor α_{auβen} and the coefficient of expansion of the glass α_{Glas} is at least 2 ppm/K, preferably at least 4 ppm/K in the temperature range from 20°C to the glass transformation temperature and the coefficient of expansion of the outer conductor α_{auβen} is greater than the coefficient of expansion α_{Glas} of the glass,
**characterized in that** the coefficient of expansion of the inner conductor α_{Innen} is in the range 4 · α_{Glas} to 1.5 · α_{Glas} , wherein the coefficient of expansion α_{Innen} of the inner conductor is preferably twice as large as the coefficient of expansion α_{Glas}.

2. The glass-metal feedthrough according to Claim 1,
**characterized in that** the outer conductor consists of a preferably nickel-free, rustproof, chemically resistant steel (stainless steel).

3. The glass-metal feedthrough according to Claim 2,
**characterized in that** the outer conductor consists of rustproof, chemically resistant steel, in particular austenitic stainless steel, in particular AISI 316 L.

4. The glass-metal feedthrough according to any one of Claims 1 to 3,
**characterized in that** the outer conductor consists of nickel-free, rustproof, chemically resistant steel, preferably a ferritic stainless steel, preferably AISI 430.

5. The glass-metal feedthrough according to any one of Claims 1 to 4, **characterized in that** the inner conductor consists of one of the following materials:
- nickel-free, ferritic stainless steel, in particular non-hardenable, ferritic stainless steel, preferably having a high chromium proportion, in particular between 10.5 and 20%, preferably 15 and 17%, preferably AISI 430
- molybdenum
- tungsten
- platinum
- niobium
- titanium
- platinum/iridium.

6. The glass-metal feedthrough according to any one of Claims 1 to 5, **characterized in that** the coefficients of expansion of inner conductor and outer conductor are selected in such a way that a joining pressure is built up on the inner conductor of at least 30 MPa, preferably of at least 50 MPa, in particular of at least 100 MPa.

7. A use of the glass-medical feedthrough according to any one of Claims 1 to 6 in implantable medical appliances or devices.

8. An element, which is introducible into the human or animal body or cell cultures containing living biological cells or attachable thereon, having a glass-metal feedthrough according to any one of Claims 1 to 6, wherein the outer conductor and the inner conductor, at least in their surface regions which come into contact with the human or animal body in the operating state, consist of metals which have a reduced allergy potential.

9. The element according to Claim 8,
**characterized in that** the metal of the outer conductor and the inner conductor in contact with the human or animal body or the cell cultures does not deposit nickel and/or chromium.

10. The element according to at least one of Claims 8 to 9, **characterized in that** the metal of the outer conductor and of the inner conductor, at least in their surface regions which come into contact in the operating state with the human or animal body or biological cells of the cell culture, consist of nickel-free and/or chromium-free steels.

11. The element according to at least one of Claims 8 to 10, **characterized in that** the metal of the inner conductor, at least in the surface regions which come into contact in the operating state with the human or animal body or biological cells of the cell culture, is selected from the group
- of the ferritic stainless steels (AISI 4xx)
- platinum
- platinum/iridium
- niobium
- titanium
- molybdenum
- tungsten
and combinations thereof.

12. The element according to Claim 11,
**characterized in that** the metal of the outer conductor, at least in the surface regions which come into contact in the operating state with the human or animal body or biological cells of the cell culture, is selected from the group
- of the austenitic stainless steels AISI 3xxx, in particular
- AISI 316 L
- of the ferritic stainless steels AISI 4xxx, in particular
- AISI 430
- AISI 630
and combinations thereof.

## Revendications

1. Traversée en verre et métal composée d'un conducteur extérieur (9), d'un verre et d'un conducteur intérieur (7), dans laquelle le conducteur intérieur possède un coefficient de dilatation αᵢₙₜ, le verre un coefficient de dilatation αᵥₑᵣᵣₑ et le conducteur extérieur un coefficient de dilatation αₑₓₜ, le coefficient de dilatation du conducteur intérieur αᵢₙₜ étant plus grand que le coefficient de dilatation du verre αᵥₑᵣᵣₑ et la différence entre le coefficient de dilatation du conducteur extérieur αₑₓₜ et le coefficient de dilatation du verre αᵥₑᵣᵣₑ étant d'au moins 2 ppm/K, de préférence au moins 4 ppm/K dans la plage de température allant de 20 °C à la température de transformation vitreuse et le coefficient de dilatation du conducteur extérieur αₑₓₜ étant plus grand que le coefficient de dilatation αᵥₑᵣᵣₑ du verre,
**caractérisée en ce que** le coefficient de dilatation du conducteur intérieur αᵢₙₜ est compris entre 4 × αᵥₑᵣᵣₑ et 1,5 × αᵥₑᵣᵣₑ, le coefficient de dilatation αᵢₙₜ du conducteur intérieur étant de préférence deux fois plus grand que le coefficient de dilatation αᵥₑᵣᵣₑ.

2. Traversée en verre et métal selon la revendication 1, **caractérisée en ce que** le conducteur extérieur est fait d'un acier de préférence sans nickel, inoxydable et résistant aux agents chimiques (acier fin).

3. Traversée en verre et métal selon la revendication 2, **caractérisée en ce que** le conducteur extérieur est fait d'un acier inoxydable et résistant aux agents chimiques, en particulier d'acier fin austénitique, en particulier d'acier AISI 316 L.

4. Traversée en verre et métal selon l'une des revendications 1 à 3, **caractérisée en ce que** le conducteur extérieur est fait d'un acier sans nickel, inoxydable et résistant aux agents chimiques, de préférence d'un acier fin ferritique, de préférence d'acier AISI 430.

5. Traversée en verre et métal selon l'une des revendications 1 à 4, **caractérisée en ce que** le conducteur intérieur se compose de l'un des matériaux suivants :
- acier fin ferritique sans nickel, en particulier acier fin ferritique non trempable, contenant de préférence une forte proportion de chrome, en particulier entre 10,5 et 20 %, de préférence entre 15 et 17 %, de préférence de l'AISI 430,
- molybdène,
- tungstène,
- platine,
- niobium,
- titane,
- platine/iridium.

6. Traversée en verre et métal selon l'une des revendications 1 à 5, **caractérisée en ce que** le coefficient de dilatation du conducteur intérieur et du conducteur extérieur est choisi de telle manière qu'une pression d'assemblage d'au moins 30 MPa, de préférence d'au moins 50 MPa, en particulier d'au moins 100 MPa, s'exerce sur le conducteur intérieur.

7. Utilisation de la traversée en verre et métal selon l'une des revendications 1 à 6 dans des appareils ou dispositifs médicaux implantables.

8. Élément pouvant être introduit dans ou appliqué sur le corps humain ou animal ou des cultures cellulaires contenant des cellules biologiques vivantes, muni d'une traversée en verre et métal selon l'une des revendications 1 à 6, dans lequel le conducteur extérieur et le conducteur intérieur se composent, au moins dans leurs zones de surface venant en contact, en fonctionnement, avec le corps humain ou animal, de métaux présentant un potentiel allergisant réduit.

9. Élément selon la revendication 8, **caractérisé en ce que** le métal du conducteur extérieur et du conducteur intérieur ne libère pas de nickel ni de chrome au contact du corps humain ou animal ou des culture cellulaires.

10. Élément selon une au moins des revendications 8 à 9, **caractérisé en ce que** le métal du conducteur extérieur et du conducteur intérieur se compose, au moins dans leurs zones de surface venant en contact, en fonctionnement, avec le corps humain ou animal ou des cellules biologiques de la culture cellulaire, d'aciers sans nickel et/ou sans chrome.

11. Élément selon une au moins des revendications 8 à 10, **caractérisé en ce que** le métal du conducteur intérieur est choisi, au moins dans les zones de surface venant en contact, en fonctionnement, avec le corps humain ou animal ou des cellules biologiques de la culture cellulaire, parmi le groupe comprenant :
- les aciers fins ferritiques (AISI 4xx),
- le platine,
- le platine/iridium,
- le niobium,
- le titane,
- le molybdène,
- le tungstène,
ainsi que des combinaisons de ceux-ci.

12. Élément selon la revendication 11, **caractérisée en ce que** le métal du conducteur extérieur est choisi, au moins dans les zones de surface venant en contact, en fonctionnement, avec le corps humain ou animal ou des cellules biologiques de la culture cellulaire, parmi le groupe comprenant :
- les aciers fins austénitiques AISI 3xxx, en particulier
- AISI 316 L,
- les aciers fins ferritiques AISI 4xx, en particulier
- AISI 430,
- AISI 630,
ainsi que des combinaisons de ceux-ci.
